(19) Europäisches Patentamt
European Patent Office
Office européen des brevets

(11) Veröffentlichungsnummer: **0 023 539**
**B1**

# (12) EUROPÄISCHE PATENTSCHRIFT

(45) Veröffentlichungstag der Patentschrift: **12.01.83**

(51) Int. Cl.³: **C 07 D 285/12**, C 07 D 417/12, C 07 D 401/12, A 01 N 47/38

(21) Anmeldenummer: **80102723.6**

(22) Anmeldetag: **16.05.80**

(54) **3-(N-1,3,4-Thiadiazolyl-2)-amino-alkyl-acrylsäurealkylester, Verfahren zu ihrer Herstellung und ihre Verwendung als Pflanzenbakterizide.**

(30) Priorität: **18.05.79 CH 4670/79**
**14.04.80 CH 2857/80**

(43) Veröffentlichungstag der Anmeldung: **11.02.81 Patentblatt 81/6**

(45) Bekanntmachung des Hinweises auf die Patenterteilung: **12.01.83 Patentblatt 83/2**

(84) Benannte Vertragsstaaten: **AT BE CH DE FR GB IT LI NL**

(56) Entgegenhaltungen: **DE-A-2 625 285**

(73) Patentinhaber: **CIBA-GEIGY AG, Patentabteilung Postfach, CH-4002 Basel (CH)**

(72) Erfinder: **Gätzi, Karl, Dr., Realpstrasse 69, CH-4054 Basel (CH)**
Erfinder: **Baumann, Hanspeter, Klusweg 98, CH-4153 Reinach (CH)**
Erfinder: **Kunz, Walter, Dr., 79 Gleneagles Road, Flixton, Manchester M31 2SA (GB)**
Erfinder: **Gloor, Bernhard, Dr., Höhenweg 10, CH-4133 Pratteln (CH)**

(74) Vertreter: **Zumstein, Fritz sen., Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# 3-(N-1,3,4-Thiadiazolyl-2)-amino-alkyl-acrylsäurealkylester, Verfahren zu ihrer Herstellung und ihre Verwendung als Pflanzenbakterizide.

Die vorliegende Erfindung betrifft Verbindungen der Formel I

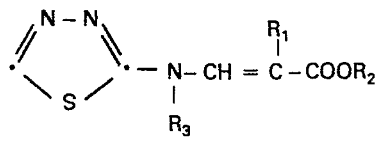 (I)

worin

$R_1$ Wasserstoff oder $C_1$–$C_4$-Alkyl,

$R_2$ $C_1$–$C_4$-Alkyl,

$R_3$ Wasserstoff oder die Gruppe $-CO-R_4$ bedeuten, wobei

$R_4$ für Alkoxyalkyl, $C_3$–$C_6$-Cycloalkyl, ein- oder mehrfach durch Halogen substituiertes oder unsubstituiertes $C_1$–$C_4$-Alkyl oder $C_2$–$C_4$-Alkenyl; oder ein ein- bis vierfach durch $C_1$–$C_4$-Alkyl, Halogen, Cyano, Nitro, Alkoxy, Alkylthio, Alkylsulfonyl oder Amino substituiertes oder unsubstituiertes Furyl, Thienyl, Pyridyl oder Phenyl steht.

Unter Alkyl oder Alkylanteil eines anderen Substituenten sind je nach Zahl der angegebenen Kohlenstoffatome beispielsweise folgende Gruppen zu verstehen: Methyl, Äthyl, Propyl, Butyl, Pentyl, Hexyl, Heptyl, Octyl usw. sowie ihre Isomeren, wie z.B. Isopropyl, Isobutyl, tert.-Butyl, Isopentyl usw. Alkenyl steht z.B. für Propenyl(1), Allyl, Butenyl-(1), Butenyl-(2), oder Butenyl-(3). Unter Cycloalkyl soll beispielsweise Cyclopropyl, Cyclobutyl, Cyclopentyl oder Cyclohexyl verstanden werden. Halogen steht z.B. für Fluor, Chlor, Brom oder Jod.

Verbindungen der Formel I sind stabile Substanzen; sie zeigen ein sehr wertvolles Mikrobizid-Spektrum und wirken insbesondere gegen phytopathogene Mikroorganismen, vor allem gegen pflanzenschädigende Bakterien.

Eine interessante Gruppe von Bakteriziden besteht aus Verbindungen der Formel I, worin $R_3$ für Wasserstoff steht.

Folgende Einzelverbindungen der Formel I sind auf Grund ihrer sehr guten bakteriziden Wirkung besonders bevorzugt:

3-(N-1,3,4-Thiadiazolyl-2)-amino-2-methylacrylsäuremethylester,

3-(N-1,3,4-Thiadiazolyl-2)-amino-2-methyl-acrylsäureethylester

3-(N-1,3,4-Thiadiazolyl-2)-amino-2-methyl-acrylsäure-n-propylester,

3-(N-1,3,4-Thiadiazolyl-2)-amino-2-ethyl-acrylsäureethylester,

3-(N-1,3,4-Thiadiazolyl-2)-amino-2-isopropylacrylsäuremethylester.

Die Verbindungen der Formel I können, wie nachfolgend im einzelnen aufgeführt, hergestellt werden. Dabei können gebildete Zwischenprodukte vor der Weiterverarbeitung isoliert, die Reaktion jedoch auch im sogenannten Eintopfverfahren durchgeführt werden. In den Formeln II, III und IV sowie $R_4COOH$ haben die Substituenten $R_1$, $R_2$, $R_3$ und $R_4$ die unter Formel I angegebenen Bedeutungen. X steht für eine der üblichen Abgangsgruppen wie z.B. Alkoxy, Benzolsulfonyloxy, p-Brombenzolsulfonyloxy, p-Tosyloxy, Niederalkylsulfonyloxy wie Mesyloxy oder insbesondere für Halogen wie Fluor, Chlor, Brom, Jod, bevorzugt für Chlor oder Brom.

Die Verbindungen der Formel I werden erfindungsgemäss hergestellt, indem man wahlweise entweder

a) 2-Amino-1,3,4-thiadiazol der Formel II

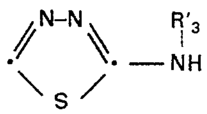 (II)

worin $R_3'$ Wasserstoff bedeutet, mit einer Verbindung der Formel III

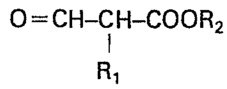 (III)

unter Wasserabspaltung kondensiert und anschliessend, sofern gewünscht, das kondensierte Produkt mit einer Säure $R_3COOH$ oder bevorzugt mit einem reaktionsfähigen Säurederivat N-acyliert oder

b) indem man eine Verbindung der Formel II, worin $R_3'$ die gleiche Bedeutung wie $R_3$ hat, mit einer Verbindung der Formel IV,

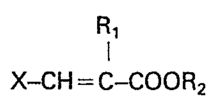 (IV)

vorzugsweise in Gegenwart einer Base und unter Abspaltung einer Verbindung der Formel H–X zur Reaktion bringt und, sofern gewünscht, das Reaktionsprodukt, im Falle $R_3'=H$, wie unter a) beschrieben, N-acyliert.

Alle Reaktionsschritte werden vorteilhaft in Gegenwart eines reaktions-inerten organischen Lösungsmittels oder Verdünnungsmittels durchgeführt. Als Verdünnungsmittel eignet sich in manchen Fällen auch der Überschuss eines Reaktanden, bei der N-Acylierung beispielsweise das überschüssige Acylierungsmittel.

Als Reaktionsmedium eignen sich für die genannten Reaktionen der Verbindungen der Formel III und IV beispielsweise aliphatische und aromatische Kohlenwasserstoffe wie Benzol, Toluol, Xylole, Petrolether; halogenierte Kohlenwasserstoffe wie Chlorbenzole, Tetrachlorkohlenstoff, Tetrachlorethylen; Ester wie Ethylacetat, Propylacetat, Butylacetat; Ether und etherartige Verbindungen wie Dialkylether (Diisopropylether, tert.-Butylmethylether usw.), Anisol, Dioxan, Dimethoxyethan; Alkohole wie Ethanol, Propanol, Butanol sowie Methylcellosolve; Nitrile wie Acetonitril, Propionitril usw. oder Gemische solcher Lösungsmittel untereinander.

Bei der Kondensationsreaktion kann sich die Gegenwart eines sauren, basischen oder neutralen Kondensations- oder Bindemittels günstig erweisen. Als Kondensationsmittel eignen sich z.B. starke Säuren wie Sulfonsäuren (p-Toluolsulfonsäure, Benzolsulfonsäure), Phosphorsäure; Amine wie Pyridin, 4-Dimethylaminopyridin, 4-Pyrollidylpyridin, Piperidin, Triethylamin, Triethylendiamin, N,N-Dimethylanilin; Anhydride wie Acetanhydrid sowie Molekularsiebe, aber auch Verbindungen wie Dicyclohexylcarbodiimid.

Die Reaktionstemperatur liegt bei der Kondensationsreaktion zum Enamin üblicherweise zwischen 0° und 180 °C, vorzugsweise zwischen 50° und 150 °C oder am Siedepunkt des Lösungsmittels oder Lösungsmittelgemisches.

Die Reaktionen mit Acrylsäurederivaten der Formel IV erfolgen vorteilhafterweise in einem aprotischen Lösungsmittel in Gegenwart einer starken Base. Als Basen eignen sich Oxyde, Hydroxide, Hydride, Carbonate und Hydrocarbonate von Alkali- und Erdalkalimetallen, Alkaliacetate sowie Organometall-Verbindungen wie Butyllithium.

Zur Beschleunigung der Reaktionsgeschwindigkeit kann bei dieser Durchführungsart die Gegenwart eines Phasentransfer-Katalysators von Vorteil sein. Beispiele derartiger Katalysatoren sind: Tetraalkylammoniumhalogenide, -hydrogensulfate und -hydroxide wie Tetrabutylammoniumchlorid, -bromid, -jodid; Triethylbenzylammoniumchlorid, -bromid; Tetrapropylammoniumchlorid, -bromid, -jodid; usw. Als Phasentransfer-Katalysatoren kommen auch Phosphonium-Salze in Betracht.

Die genannten N-Acylierungs-Reaktionen erfolgen entweder durch Umsetzung mit der Säure $R_4COOH$ selbst, vorteilhafterweise jedoch mit einem reaktionsfähigen Säurederivat wie Ester, Säureanhydrid oder Säurehalogenid, bevorzugt Säurechlorid oder Säurebomid.

Bei der Acylierung kann die Gegenwart eines Reaktionskatalysators wie Dimethylformamid von Vorteil sein.

Die Reaktionstemperaturen liegen zwischen 0° und 180 °C, vorzugsweise 0° und 150 °C bzw. am Siedepunkt des Lösungsmittels bzw. Lösungsmittelgemisches. In manchen Fällen ist die Verwendung von säurebindenden Mitteln bzw. Kondensationsmitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z.B. Stickstoff, aus dem Reaktionsgemisch vertrieben oder mit Hilfe von Molekularsieben gebunden werden.

Alle Reaktionsschritte werden vorzugsweise bei Normaldruck durchgeführt.

Die Ausgangsstoffe der Formeln II, III und IV sind allgemein bekannt oder werden nach an sich bekannten Methoden hergestellt.

Das beschriebene Verfahren ist in allen Teilschritten ein Bestandteil der Erfindung.

Aus der US Patentschrift Nr. 3 891 762 sind verwandte 2-Amino-1,3,4-thiadiazol-Derivate bekannt, darunter auch der Ausgangsstoff der Formel II. Ihre pflanzenbakterizide Wirkung wird ebenfalls beschrieben. Es hat sich aber erwiesen, dass die in dieser Patentschrift genannten Verbindungen eine teratogene Wirkung aufweisen (vgl. Teratology 7, 65 (1973); 9, 179 (1974) und 10, 90 (1964). Diese schwerwiegende Nebenwirkung tritt überraschenderweise bei den erfindungsgemässen Verbindungen nicht auf (vgl. biologische Beispiele). Ausserdem sind einige der bekannten Verbindungen im Gegensatz zu den erfindungsgemässen Stoffen phytotoxisch.

Es wurde nun überraschend gefunden, dass Verbindungen der Formel I ein für praktische Bedürfnisse sehr günstiges Mikrobizid-Spektrum aufweisen. Ihr Haupteinsatzgebiet wird in der Bekämpfung von schädlichen Mikroorganismen, vor allem von phytopathogenen Bakterien gesehen. Verbindungen der Formel I lassen sich insbesondere zum Schutz von Kulturpflanzen einsetzen ohne diese durch unerwünschte Nebenwirkungen wie Phytotoxizität zu beeinflussen. Kulturpflanzen seien im Rahmen vorliegender Erfindung beispielsweise Getreidekulturen: Reis; Citrusfrüchte; Baumwolle; Gemüse (z.B. Tomaten, Paprika, Kohl, Kartoffeln, Rüben etc.); Walnussbäume; Obstbäume (z.B. Prunus-Arten, Äpfel, Birnen, Kirschen); Zierpflanzen (z.B. Geranien, Begonien und viele andere).

Diese Aufzählung stellt nur die wichtigsten und meistgefährdeten Kulturpflanzen vor. Verwandte Arten oder vergesellschaftete Pflanzen sind selbstverständlich mit eingeschlossen.

Die Verbindungen der Formel I können vor allem gegen Xanthomonaden (z.B. Xanthomonas ovyzae, X. citri, X. campestis, X. malvacearum etc.), aber auch gegen phytopathogene Arten der Gattungen Pseudomonas, Erwinia, Agrobakterium und Corynebakterium eingesetzt werden, und zwar in allen Bereichen, in denen sich derartige Erreger aufhalten und entwickeln, unter Einschluss von Wirtspflanzen aus der Unkrautflora.

Mit den Wirkstoffen der Formel I können an Pflanzen oder Pflanzenteilen (Früchte, Blüten, Laubwerk, Stengel, Knollen, Wurzeln) von Nutzkulturen die auftretenden Bakterien eingedämmt oder vernichtet werden, wobei auch später zuwachsende Pflanzenteile von derartigem Befall verschont bleiben.

Überdies wirken die Verbindungen der Formel I systemisch.

Das Vorliegen einer Doppelbindung im Molekül hat die Existenz von cis/trans Isomeren zur Folge, die sich in ihren biologischen Eigenschaften unterscheiden. Ausgeprägte Bakterizide sind diejenigen Verbindungen, bei denen Thiadiazolylaminogruppe und Carboxylgruppe trans-ständig zueinander stehen.

Die Substanzen der Formel I können für sich allein oder zusammen mit geeigneten Trägern und/oder Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen. Lösungs-, Dispergier-, Netz-, Haft-, Verdikkungs-, Binde- oder Düngemitteln. Die Herstellung solcher Mittel erfolgt in an sich bekannter Weise durch inniges Vermischen und Vermahlen der Bestandteile. Zur Applikation können die Verbindungen der Formel I in den folgenden Aufarbeitungsformen vorliegen (wobei die Gewichtsprozentangaben vorteilhafte Mengen an Wirkstoff darstellen).

Feste Aufarbeitungsformen:

Stäubemittel, Streumittel (bis 10%) Granulate, Umhüllungsgranulate, Imprägnierungsgranulate und Homogengranulate, Pellets (Körner) (1 bis 80%).

Flüssige Aufarbeitungsformen:

a) in Wasser dispergierbare Wirkstoffkonzentrate:

Spritzpulver (wettable powders) Pasten 25–90% in der Handelspackung, 0,01 bis 15% in gebrauchsfertiger Lösung, Emulsionen, Lösungskonzentrate (10 bis 50%); (0,01 bis 15% in gebrauchsfertiger Lösung)

b) Lösungen, Aerosole,

Der Gehalt an Wirkstoff in den oben beschriebenen Mitteln liegt zwischen 0,1 und 95 Gewichtsprozent. Solche Mittel sind gleichfalls Gegenstand dieser Erfindung.

Die Verbindungen der Formel I können, um sie den gegebenen Umständen anzupassen, selbstverständlich zur Verbreiterung des Wirkungsspektrums mit anderen geeigneten Pestiziden, wie z.B. Fungiziden, Bakteriziden, Insektiziden, Akariziden, Herbiziden oder den Pflanzenwuchs beeinflussenden Wirkstoffen zusammen eingesetzt werden.

Die Erfindung betrifft somit ferner die Verwendung der Verbindungen der Formel I zur Bekämpfung phytopathogener Bakterien.

Die nachfolgenden Beispiele dienen zur näheren Erläuterung der Erfindung, ohne dieselbe einzuschränken. Die Temperaturen sind in Celsiusgraden angegeben. Prozente und Teile beziehen sich auf Gewicht.

Herstellungsbeispiele:

Beispiel 1

Herstellung von 3-(N-1,3,4-Thiadiazolyl-2)-amino-2-methylacrylsäuremethylester der Formel

(Verb. No. 1)

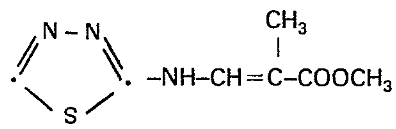

303 g (3,0 Mol) 2-Amino-1,3,4-thiadiazol wurden in 2 Liter Methylcellosolve zusammen mit 352,8 g (3,18 Mol) α-Formyl-propionsäuremethylester 16 Std. bei 120° gehalten. Dann wurde abgekühlt, die ausgefallenen Kristalle abgesaugt und aus Äthanol umkristallisiert. Die so erhaltenen weissen Kristalle schmolzen bei 176–179°. Durch Eindampfen der Mutterlauge und erneute Umkristallisation konnte eine weitere Menge der Verb. No. 1 gewonnen werden.

Beispiel 2

Herstellung von 3-(N-1,3,4-thidiazolyl-2-N-cyclopropylcarbonyl)-amino-2-methylacrylsäuremethylester der Formel

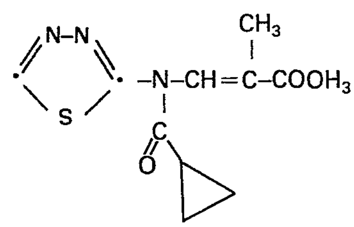

(Verb. No. 17)

16,6 g (0,83 Mol) 3-(N-1,3,4-Thiadiazolyl-2-)-amino-2-methyl-acrylsäuremethylester (Verb. No. 1) wurden in 200 ml Toluol gegeben und mit 10,35 g (0,1 Mol) Cyclopropancarbonsäurechlorid versetzt. Das Gemisch wurde 18 Stunden unter Rückfluss erhitzt, mit etwas Ativkohle versetzt und heiss filtriert. Nach Zugabe von Petrolether kristallisiert ein Rohprodukt, das nach Umkristallisation aus Ethanol einen Schmelzpunkt von 116–118 °C aufwies.

Analog zu den Beispielen 1 und 2 können folgende Verbindungen hergestellt werden:

Tabelle 1:

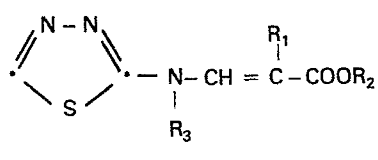

(I)

| Verb. No. | $R_1$ | $R_2$ | $R_3$ | phys. Konst. |
|---|---|---|---|---|
| 1 | $CH_3$ | $CH_3$ | H | Smp. 176–179° |
| 2 | $C_2H_5$ | $CH_3$ | H | Smp. 186–188° |
| 3 | n–$C_3H_7$ | $CH_3$ | H | Smp. 131–134° |
| 4 | n–$C_4H_9$ | $CH_3$ | H | Smp. 143–145° |
| 5 | H | $CH_3$ | H | |
| 6 | $CH_3$ | $C_2H_5$ | H | Smp. 176–179° |
| 7 | $CH_3$ | n–$C_3H_7$ | H | Smp. 157–160° |
| 8 | $CH_3$ | n–$C_4H_9$ | H | Smp. 132–133° |
| 9 | $C_2H_5$ | $C_2H_5$ | H | Smp. 162–170° |
| 10 | $CH_3$ | t–$C_4H_9$ | H | |
| 11 | i–$C_3H_7$ | $CH_3$ | H | Smp. 138–142° |
| 12 | n–$C_3H_7$ | $C_2H_5$ | H | Smp. 145–147° |
| 13 | $CH_3$ | $CH_3$ | CO–(2-Furyl) | Smp. 118–120° |
| 14 | $CH_3$ | n–$C_3H_7$ | CO–$CH_2$–Cl | |
| 15 | $CH_3$ | $CH_3$ | CO–$CH_2OCH_3$ | Smp. 119–121° |
| 16 | $CH_3$ | $CH_3$ | CO–$CH_3$ | Smp. 153–157° |
| 17 | $CH_3$ | $CH_3$ | CO-Cyclopropyl | Smp. 116–118° |
| 18 | $C_2H_5$ | $CH_3$ | CO-Cyclopropyl | |
| 19 | $CH_3$ | $CH_3$ | CO–$C_6H_4$(4–$CH_3$) | Smp. 128–132° |
| 20 | $CH_3$ | $CH_3$ | CO–$C_6H_3$($Cl_2$ 2,4) | Smp. 105–109° |
| 21 | $CH_3$ | $CH_3$ | CO–$CH_2Cl$ | Smp. 170–175° |
| 22 | $CH_3$ | i–$C_3H_7$ | CO–(2-Thienyl) | |
| 23 | $CH_3$ | $CH_3$ | CO–CH=$CH_2$ | |
| 24 | n–$C_3H_7$ | $CH_3$ | CO–$C_6H_3$(3–$NO_2$)(4–Cl) | |
| 25 | $CH_3$ | $CH_3$ | CO–CCl=$CCl_2$ | |
| 26 | $CH_3$ | $CH_3$ | CO–$CH_2CH_3$ | |
| 27 | $CH_3$ | $CH_3$ | CO-Cyclohexyl | |
| 28 | $CH_3$ | $CH_3$ | CO– 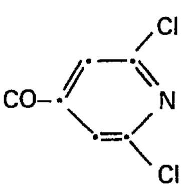 | Smp. 140–146° |
| 29 | $CH_3$ | $CH_3$ | CO–$C_6H_4$(3–Cl) | Smp. 124–130° |

Formulierungsbeispiele

Beispiel 3

Stäubemittel: Zur Herstellung eines a) 5%igen, b) 2%igen und c) 80%igen Stäubemittels werden die folgenden Stoffe verwendet:

a) 5 Teile Wirkstoff
95 Teile Talkum;

b) 2 Teile Wirkstoff
1 Teil hochdisperse Kieselsäure,
97 Teile Talkum;

c) 80 Teile Wirkstoff
17 Teile Talkum
3 Teile hochdisperse Kieselsäure

Die Wirkstoffe werden mit den Trägerstoffen vermischt und vermahlen und können in dieser Form zur Anwendung verstäubt werden.

Beispiel 4

Granulat: Zur Herstellung eines 5%igen Granulates werden die folgenden Stoffe verwendet:

5 Teile Wirkstoff
0,25 Teile epoxidiertes Pflanzenöl,
0,25 Teile Cetylpolyglykoläther,
3,50 Teile Polyäthylenglykol
91 Teile Kaolin (Korngrösse 0,3–0,8 mm).

Die Aktivsubstanz wird mit epoxidiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft.

Beispiel 5

Spritzpulver: Zur Herstellung eines a) 70%igen b) 40%igen c) und d) 25%igen e) 10%igen Spritzpulvers werden folgende Bestandteile verwendet:

a) 70 Teile Wirkstoff
5 Teile Natriumdibutylnaphthylsulfonat,
3 Teile Naphthalinsulfonsäuren-Phenolsulfonsäuren-Formaldehyd-Kondensat 3:2:1,

10 Teile Kaolin
12 Teile Champagne-Kreide;

b) 40 Teile Wirkstoff
5 Teile Ligninsulfonsäure-Natriumsalz,
1 Teil Dibutylnaphthalinsulfonsäure-Natriumsalz,
54 Teile Kieselsäure;

c) 25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalin-sulfonat,
19,5 Teile Kieselsäure,
19,5 Teile Champagne-Kreide,
28,1 Teile Kaolin;

d) 25 Teile Wirkstoff
2,5 Teile Isooctylphenoxy-polyoxy-äthylenäthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff
3 Teile Gemisch der Natriumsalze von gesättigten Fettalkoholsulfaten,
5 Teile Naphthalinsulfonsäure/Formaldehyd-Kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 6

Emulgierbare Konzentrate: Zur Herstellung eines 25%igen emulgierbaren Konzentrates werden folgende Stoffe verwendet:

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Anwendungskonzentration hergestellt werden, die besonders zur Blattapplikation geeignet sind.

Die Wirkstoffe der Formel I können auch in Form solcher Dispersionskonzentrate mit Hilfe von feinzerteilenden Sprühgeräten appliziert werden.

Biologische Beispiele

Beispiel 7:

Wirkung gegen Xanthomonas oryzae auf Reis

a) Residual-protektive Wirkung

Reispflanzen der Sorte «Caloro» oder «S6» wurden nach 3-wöchiger Anzucht im Gewächtshaus mit der Prüfsubstanz in Form einer Spritzbrühe (0,06% Aktivsubstanz) besprüht. Nach eintätigem Antrocknen dieses Spritzbelages wurden die Pflanzen in einem Klimaraum bei 24 °C und 75–85% relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der residualen Wirksamkeit der Prüfsubstanz, dabei bewirkten die Verbindungen Nr. 1, 2, 3, 4, 6, 7, 8, 9, 11, 12, 13 und 15, dass weniger als 20% der Pflanzen befallen wurden. Als Vergleich wurden unbehandelte, aber infizierte Kontrollpflanzen gewählt, die einen Befall von 100% aufweisen.

b) Systemische Wirkung

Reispflanzen der Sorte «Caloro» der «S6» wurden nach 3-wöchiger Anzucht im Gewächshaus mit einer Suspension der Prüfsubstanz begossen (0,006% Aktivsubstanz bezogen auf das Erdvolumen). Drei Tage nach dieser Behandlung wurden die Pflanzen in einem Klimaraum bei 24 °C und 75–85% realtiver Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nektrotisch. Das Ausmass dieser Krankheitssymptome diente zur Beurteilung der systemischen Wirksamkeit der Prüfsubstanz. Verbindungen der Formel I, darunter die der Nr. 1, 2, 3, 4, 6, 7, 8, 9, 11, 12, 13 und 15, erreichten in diesem Versuch eine Verminderung des Befalls auf weniger als 20% im Vergleich zu unbehandelten, aber infizierten Kontrollpflanzen (100% Befall).

Beispiel 8

Wirkung gegen Xanthomonas vesicatoria auf Paprika

a) Residual-protektive Wirkung

Paprikapflanzen der Sorte «California Wonder» wurden nach 3-wöchiger Anzucht im Gewächshaus mit der Prüfsubstanz in Form einer Spritzbrühe (0,06% Aktivsubstanz) besprüht. Nach eintägigem Antrocknen dieses Spritzbelages wurden die Pflanzen in einem Klimaraum bei 26 °C und 95–100% relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der residualen Wirksamkeit der Prüfsubstanz. Verbindungen der Formel I, darunter die der Nr. 1, 2, 4, 6, 8, 9, 12, 13 und 15, erreichten in diesem Versuch eine Verminderung des Befalls auf weniger als 20% im Vergleich zu unbehandelten, aber infizierten Kontrollpflanzen (100% Befall).

b) Systemische Wirkung
Paprikapflanzen der Sorte «California Wonder» wurden nach 3-wöchiger Anzucht im Gewächshaus mit einer Suspension der Prüfsubstanz begossen (0,006% Aktivsubstanz bezogen auf das Erdvolumen). Drei Tage nach dieser Behandlung wurden die Pflanzen in einem Klimaraum bei 26 °C und 95–100% relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der systemischen Wirksamkeit der Prüfsubstanz.

In obigem Versuch bewirkten Verbindungen der Formel I, darunter die der Nr. 1, 2, 4, 7, 9, 11 und 15, eine Verminderung des Befalls auf weniger als 20% im Vergleich zu unbehandelten, aber infizierten Kontrollpflanzen (100% Befall).

Beispiel 9
Prüfung auf mögliche teratogene Wirkung
Testsubstanz

(A)

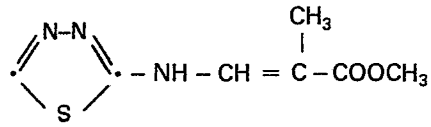

Verb. Nr. 1

(B)

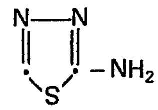

US 3 891 762

Trächtige Rattenweibchen wurden vom 6. bis zum 15. Tag der Schwangerschaft entweder

– mit 0,3%iger Lösung der Substanz A (Lösung in wässriger Natriumcarboxymethylcellulose) in einer Menge von 1 ml/100 g Körpergewicht oder

– mit 0,2%iger Lösung der Substanz B (Lösung in Natriumcarboxymethylcellulose/physiologische Kochsalzlösung) in einer Menge von 0,5 ml/100 g Körpergewicht oder nur

– mit der Trägerlösung (Kontrolle)

behandelt.

Die am 21. Tag der Gestation getöteten Tiere wurden auf die Zahl der missgebildeten lebenden Föten untersucht.

| Ergebnisse | A | Kontrolle | B | Kontrolle |
|---|---|---|---|---|
| Gesamtdosis | 30 mg/kg | – | 10 mg/kg | – |
| Embryonal- + Fötaltod in % der Implantate | 10,3 | 7,6 | 71,1 | 12,8 |
| Zahl der missgebildeten lebenden Föten/Gesamtzahl | 0/113 | 0/121 | 26/27 | 0/109 |

**Patentansprüche für die Vertragsstaaten: BE, CH, LI, DE, FR, GB, IT, NL**

1. Verbindungen der Formel I

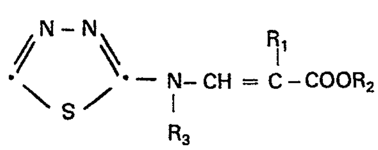

(I)

worin

$R_1$ Wasserstoff oder $C_1$–$C_4$ Alkyl und

$R_2$ $C_1$–$C_4$ Alkyl,

$R_3$ Wasserstoff oder die Gruppe –CO–$R_4$ bedeuten, wobei

$R_4$ Alkoxyalkyl, $C_3$–$C_6$-Cycloalkyl, ein ein- oder mehrfach durch Halogen substituiertes oder unsubstituiertes $C_1$–$C_4$-Alkyl oder $C_2$–$C_4$-Alkenyl; oder ein ein- bis vierfach durch $C_1$–$C_4$-Alkyl, Halogen, Cyano, Nitro, Alkoxy, Alkylthio, Alkylsulfonyl oder Amino substituiertes oder unsubstituiertes Furyl, Thienyl, Pyridyl oder Phenyl steht.

2. Verbindungen der Formel I nach Anspruch 1, worin $R_3$ Wasserstoff bedeutet.

3. Eine Verbindung aus der Gruppe
3-(N-1,3,4-Thiadiazolyl-2)-amino-2-methyl-acrylsäuremethylester,
3-(N-1,3,4-Thiadiazolyl-2)-amino-2-methyl-acrylsäureethylester,
3-(N-1,3,4-Thiadiazolyl-2)-amino-2-methyl-acrylsäure-n.proylester,
3-(N-1,3,4-Thiadiazolyl)-amino-2-ethyl-acrylsäureethylester,
3-(N-1,3,4-Thiadiazolyl-2)-amino-2-isopropyl-acrylsäuremethylester.

4. Verfahren zur Herstellung von Verbindungen der Formel I, dadurch gekennzeichnet, dass man wahlweise entweder

a) 2-Amino-1,3,4-thiadiazol der Formel II

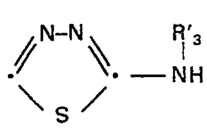

(II)

worin $R_3'$ Wasserstoff bedeutet, mit einer Verbindung der Formel III

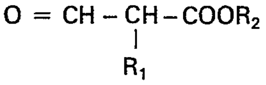

(III)

unter Wasserabspaltung kondensiert und anschliessend, sofern gewünscht, das kondensierte Produkt mit einer Säure $R_4COOH$ oder bevorzugt mit einem reaktionsfähigen Säurederivat N-acyliert, oder

b) indem man eine Verbindung der Formel II, worin $R_3'$ die gleiche Bedeutung wie $R_3$ hat, mit einer Verbindung der Formel IV

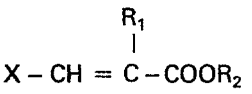

(IV)

vorzugsweise in Gegenwart einer Base und unter Abspaltung einer Verbindung der Formel H–X zur Reaktion bringt und, sofern gewünscht, das Reaktionsprodukt, im Falle $R_3'$ = Wasserstoff, wie unter a) beschrieben, N-acyliert, wobei $R_1$, $R_2$, $R_3$ und $R_4$ die unter Formel I angegebenen Bedeutungen haben und X für eine der üblichen Abgangsgruppen steht, insbesondere für Halogen.

5. Mittel zur Bekämpfung und/oder Verhütung eines Befalls durch phytopathogene Bakterien, dadurch gekennzeichnet, dass es als mindestens eine aktive Komponente eine Verbindung der Formel I gemäss Anspruch 1 zusammen mit einem oder mit mehreren pflanzenverträglichen Träger- oder Verdünnungsstoffen enthält.

6. Mittel nach Anspruch 5, dadurch gekennzeichnet, dass es als aktive Komponente eine Verbindung der Formel I gemäss Anspruch 2 enthält.

7. Mittel nach Anspruch 5, dadurch gekennzeichnet, dass es als aktive Komponente eine Verbindung der Formel I gemäss Anspruch 3 enthält.

8. Verwendung einer Verbindung der Formel I gemäss Anspruch 1 zur Eindämmung von phytopathogenen Bakterien, gekennzeichnet durch die Applikation der Aktivsubstanz auf den Lebenraum der Bakterien.

9. Verwendung nach Anspruch 8 von Verbindungen der Formel I gemäss Anspruch 2.

10. Verwendung nach Anspruch 8 von Verbindungen der Formel I gemäss Anspruch 3.

11. Verwendung nach Anspruch 8, dadurch gekennzeichnet, dass es sich bei den Bakterien um Xanthomonas Arten handelt.

**Patentansprüche für den Vertragsstaat: AT**

1. Mittel zur Bekämpfung und/oder Verhütung eines Befalls durch phytopathogene Bakterien, dadurch gekennzeichnet, dass es als mindestens eine aktive Komponente eine Verbindung der Formel

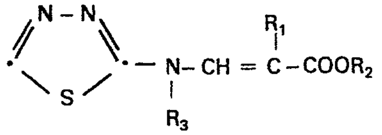

(I)

worin

$R_1$ Wasserstoff oder $C_1$–$C_4$-Alkyl und

$R_2$ $C_1$–$C_4$ Alkyl,

$R_3$ Wasserstoff oder die Gruppe –CO–$R_4$ bedeuten, wobei

$R_4$ für Alkoxyalkyl, $C_3$–$C_6$-Cycloalkyl, ein ein- oder mehrfach durch Halogen substituiertes oder unsubstituiertes $C_1$–$C_4$-Alkyl oder $C_2$–$C_4$-Alkenyl; oder ein ein- bis vierfach durch $C_1$–$C_4$-Alkyl, Halogen, Cyano, Nitro, Alkoxy, Alkylthio, Alkylsulfonyl oder Amino substituiertes oder unsubstituiertes Furyl, Thienyl, Pyridyl oder Phenyl steht zusammen mit einem oder mit mehreren pflanzenverträglichen Träger- oder Verdünnungsstoffen enthält.

2. Mittel nach Anspruch 1, dadurch gekennzeichnet, dass es als mindestens eine aktive Komponente eine Verbindung der Formel I enthält, worin $R_3$ für Wasserstoff steht und die übrigen Substituenten wie unter Formel I definiert sind.

3. Mittel nach Anspruch 1, dadurch gekennzeichnet, dass es als mindestens eine aktive Komponente eine Verbindung aus der Gruppe

3-(N-1,3,4-Thiadiazolyl-2 (-amino-2-methyl-acrylsäuremethylester,

3-(N-1,3,4-Thiadiazolyl-2)-amino-2-methyl-acrylsäureethylester,

3-(N-1,3,4-Thiadiazolyl-2)-amino-2-methyl-acrylsäure-n.propylester,

3-(N-1,3,4-Thiadiazolyl-2)-amino-2-ethyl-acrylsäureethylester und

3-(N-1,3,4-Thiadiazolyl-2)-amino-2-isopropyl-acrylsäuremethylester enthält.

4. Verfahren zur Herstellung von Verbindungen der Formel I,

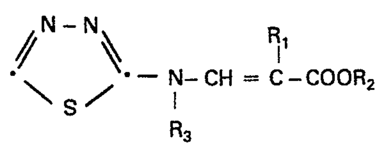

(I)

worin

$R_1$ Wasserstoff oder $C_1$–$C_4$-Alkyl und

$R_2$ $C_1$–$C_4$-Alkyl,

$R_3$ Wasserstoff oder die Gruppe –CO–$R_4$ bedeuten, wobei

$R_4$ für Alkoxyalkyl, $C_3$–$C_6$-Cycloalkyl, ein ein- oder mehrfach durch Halogen substituiertes oder unsubstituiertes $C_1$–$C_4$-Alkyl oder $C_2$–$C_4$-Alkenyl; oder ein ein- bis vierfach durch $C_1$–$C_4$-Alkyl, Halogen, Cyano, Nitro, Alkoxy, Alkylthio, Alkylsulfonyl oder Amino substituiertes oder unsubstituiertes Furyl, Thienyl, Pyridyl oder Phenyl steht, dadurch gekennzeichnet, dass man wahlweise entweder

a) 2-Amino-1,3,4-thiadiazol der Formel II

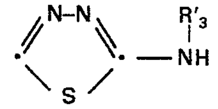

(II)

worin $R_3'$ Wasserstoff bedeutet, mit einer Verbindung der Formel III

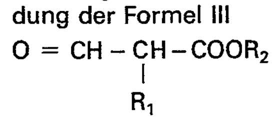

(III)

unter Wasserabspaltung kondensiert und anschliessend, sofern gewünscht, das kondensierte Produkt mit einer Säure $R_4COOH$ oder mit einem reaktionsfähigen Säurederivat N-acyliert, oder

b) indem man eine Verbindung der Formel II, worin $R_3'$ die gleiche Bedeutung wie $R_3$ hat, mit einer Verbindung der Formel IV

$$X-CH=\underset{}{\overset{R_1}{\overset{|}{C}}}-COOR_2 \qquad (IV)$$

vorzugsweise in Gegenwart einer Base und unter Abspaltung einer Verbindung der Formel H–X zur Reaktion bringt und, sofern gewünscht, das Reaktionsprodukt, im Falle $R_3'$ = Wasserstoff, wie unter a) beschrieben, N-acyliert, wobei $R_1$, $R_2$, $R_3$ und $R_4$ die unter Formel I angegebenen Bedeutungen haben und X für eine der üblichen Abgangsgruppen steht.

5. Verwendung einer Verbindung der Formel I

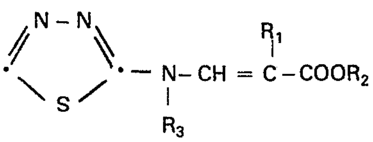

(I)

worin

$R_1$ Wasserstoff oder $C_1$–$C_4$-Alkyl und

$R_2$ $C_1$–$C_4$-Alkyl,

$R_3$ Wasserstoff oder die Gruppe –CO–$R_4$ bedeuten, wobei

$R_4$ für Alkoxyalkyl, $C_3$–$C_6$-Cycloalkyl, ein ein- oder mehrfach durch Halogen substituiertes $C_1$–$C_4$-Alkyl oder $C_2$–$C_4$-Alkenyl; oder ein ein- bis vierfach durch $C_1$–$C_4$-Alkyl, Halogen, Cyano, Nitro, Alkoxy, Alkylthio, Alkylsulfonyl oder Amino substituiertes oder unsubstituiertes Furyl, Thienyl, Pyridyl oder Phenyl steht, zur Eindämmung von phytopathogenen Bakterien, gekennzeichnet durch die Applikation der Aktivsubstanz auf den Lebensraum der Bakterien.

6. Verwendung nach Anspruch 5, dadurch gekennzeichnet, dass der Substituent $R_3$ für Wasserstoff steht und die übrigen Substituenten wie unter Formel I definiert sind.

7. Verwendung nach Anspruch 5, dadurch gekennzeichnet, dass man eine Verbindung der Formel I aus der Gruppe

3-(N-1,3,4-Thiadiazolyl-2)-amino-2-methyl-acrylsäuremethylester,

3-(N-1,3,4-Thiadiazolyl-2)-amino-2-methyl-acrylsäureethylester,

3-(N-1,3,4-Thiadiazolyl-2)-amino-2-methyl-acrylsäure-n.propylester,

3-(N-1,3,4-Thiadiazolyl-2)-amino-2-ethyl-acrylsäureethylester und

3-(N-1,3,4-Thiadiazolyl-2)-amino-2-isopropyl-acrylsäuremethylester einsetzt.

8. Verwendung nach Anspruch 5, dadurch gekennzeichnet, dass es sich bei den Bakterien um Xanthomonas Arten handelt.

**Revendications pour les Etats contractant: BE, CH, LI, DE, FR, GB, IT, NL**

1. Composés de formule I

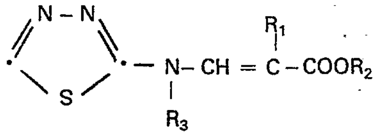

(I)

dans laquelle

$R_1$ représente l'hydrogène ou un groupe alkyle en $C_1$–$C_4$ et

$R_2$ représente un groupe alkyle en $C_1$–$C_4$;

$R_3$ représente l'hydrogène ou le groupe –CO–$R_4$,

$R_4$ représente un groupe alcoxyalkyle, cycloalkyle en $C_3$–$C_6$, un groupe alkyle en $C_1$–$C_4$ ou alcényle en $C_2$–$C_4$ nonsubstitué ou mono- ou polysubstitué par des halogènes; ou un groupe furyle, thiényle, pyridyle ou phényle non substitué ou mono- à tétra- substitué par des groupes alkyles en $C_1$–$C_4$, des halogènes, des groupes cyano, nitro, alcoxy, alkylthio, alkylsulfonyle ou amino.

2. Composés de formule I selon la revendication 1, dans laquelle $R_3$ représente l'hydrogène.

3. un composé du groupe:

3-(n-1,3,4-thiadiazolyl-2)-amino-2-méthyl-acrylate de méthyle

3-(N-1,3,4-thiadiazolyl-2)-amino-2-méthyl-acrylate d'éthyle

3-(N-1,3,4-thiadiazolyl-2)-amino-2-méthyl-acrylate de n-propyle

3-(N-1,3,4-thiadiazolyl-2)-amino-2-éthyl-acrylate d'éthyle,

3-(N-1,3,4-thiadiazolyl-2)-amino-2-isopropyl-acrylate de méthyle.

4. Procédé de préparation des composés de formule I, caractérisé en ce que, au choix,

a) ou bien on condense un 2-amino-1,3,4-thiadiazole de formule II

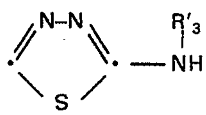

(II)

dans laquelle $R_3'$ représente l'hydrogène, avec un composé de formule III

$$O=CH-\underset{R_1}{\underset{|}{CH}}-COOR_2 \qquad (III)$$

avec séparation d'eau et ensuite, si on le désire, on soumet le produit de condensation à N-acylation par un acide $R_4COOH$ ou, de préférence, par un dérivé réactif d'acide,

b) ou bien, on fait réagir un composé de formule II dans laquelle $R_3'$ a la même signification que $R_3$, avec un composé de formule IV

$$X-CH=\overset{R_1}{\overset{|}{C}}-COOR_2 \qquad (IV)$$

de préférence en présence d'une base, et avec séparation d'un composé de formule H–X et, si on

le désire, on soumet à N-acylation comme décrit ci-dessous sous a) le produit de réaction obtenu dans le cas ou $R_3'$ représente l'hydrogène, étant précisé que $R_1$, $R_2$, $R_3$ and $R_4$ ont les significations indiquées en référence à la formule I et X représente l'un des groupes éliminables usuels, en particulier un halogène.

5. Produit pour combattre et/ou prévenir une infestation par de bactéries phytopathogènes, caractérisé en ce qu'il contient au moins un composant actif consistant en un composé de formule I selon la revendication 1 avec un ou plusieurs véhicules ou diluants tolérés par les plantes.

6. Produit selon la revendication 5, caractérisé en ce qu'il contient en tant que composant actif, un composé de formule I selon la revendication 2.

7. Produit selon la revendication 5, caractérisé en ce qu'il contient en tant que composant actif un composé de formule I selon la revendication 3.

8. Utilisation d'un composé de formule I selon la revendication 1 pour limiter la prolifération de bactéries phytopathogènes, caractérisé en ce que l'on applique la substance active sur l'habitat des bactéries.

9. Utilisation selon la revendication 8 de composés de formule I selon la revendication 2.

10. Utilisation selon la revendication 8 de composés de formule I selon la revendication 3.

11. Utilisation selon la revendication 8 caractérisée en ce que les bactéries appartiennent aux espèces Xanthomonas.

**Revendications pour l'Etat contractant: AT**

1. Produit pour combattre et/ou prévenir une infestation par les bactéries phytopathogènes, caractérisé en ce qu'il contient au moins un composant actif consistant en un composé de formule

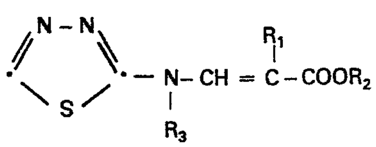

(I)

dans laquelle

$R_1$ représente l'hydrogène ou un groupe alkyle en $C_1$–$C_4$ et

$R_2$ représente un groupe alkyle en $C_1$–$C_4$;

$R_3$ représente l'hydrogène ou le groupe $-CO-R_4$,

$R_4$ représente un groupe alcoxyalkyle, cycloalkyle en $C_3$–$C_6$, un groupe alkyle en $C_1$–$C_4$ ou alcényle en $C_2$–$C_4$ non substitué ou mono- ou polysubstitué par des halogènes: ou un groupe furyle, thiényle, pyridyle ou phényle non substitué ou mono- à tétrasubstitué par des groupes alkyles en $C_1$–$C_4$, des halogènes, des groupes cyano, nitro, alcoxy, alkylthio, alkylsufonyle ou amino, avec un ou plusieurs véhicules ou diluants tolérés par les plantes.

2. Produit selon la revendication 1, caractérisé en ce qu'il contient au moins un composant actif consistant en un composé de formule I dans laquelle $R_3$ représente l'hydrogène, les autres symboles ayant les significations indiquées en référence à la formule I.

3. Produit selon la revendication 1, caractérisé en ce qu'il contient au moins un composant actif consistant en un composé du groupe:
3-(N-1,3,4-thiadiazolyl-2)-amino-2-méthyl-acrylate de méthyle,
3-(N-1,3,4-thiadiazolyl-2)-amino-2-méthyl-acrylate d'éthyle
3-(N-1,3,4-thiadiazolyl-2)-amino-2-méthyl-acrylate de n-propyle
3-(N-1,3,4-thiadiazolyl-2)-amino-2-éthyl-acrylate d'éthyle,
3-(N-1,3,4-thiadiazolyl-2)-amino-2-isopropyl-acrylate de méthyl.

4. Procédé de préparation des composés de formule I,

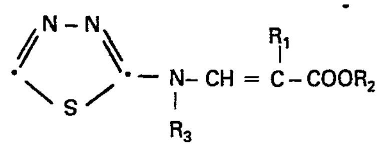

(I)

dans laquelle,

$R_1$ représente l'hydrogène ou un groupe alkyle en $C_1$–$C_4$ et

$R_2$ représente un groupe alkyle en $C_1$–$C_4$;

$R_3$ représente l'hydrogène ou le groupe $-CO-R_4$,

$R_4$ représente un groupe alcoxyalkyle, cycloalkyle en $C_3$–$C_6$; un groupe alkyle en $C_1$–$C_6$ ou alcényle en $C_2$–$C_4$ non substitué ou mono- ou polysubstitué par des halogènes; ou un groupe furyle, thiényle, pyridyle ou phényle non substitué ou mono- à tétra- substitué par des groupes cyano, nitro, alcoxy, alkylthio, alkylsulfonyle ou amino, caractérisé en ce que, au choix,

a) ou bien on condense un 2-amino-1,3,4-thiadiazole de formule II

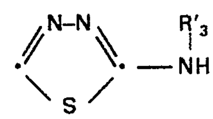

(II)

dans laquelle $R_3'$ représente l'hydrogène, avec un composé de formule III

$$O = CH - \underset{\displaystyle R_1}{\underset{|}{CH}} - COOR_2 \qquad (III)$$

avec séparation d'eau et ensuite si on le désire, on soumet le produit de condensation à N-acylation par un acide $R_4COOH$ ou, de préférence par un dérivé réactif d'acide

b) ou bien on fait réagir un composé de formule II dans laquelle $R_3'$ a la même signification que $R_3$, avec un composé de formule IV

$$X - CH = \overset{\displaystyle R_1}{\overset{|}{C}} - COOR_2 \qquad (IV)$$

de préférence en présence d'une base, et avec séparation d'un composé de formule H–X et, si on le désire, on soumet à N-acylation comme décrit ci-dessous sous a) le produit de réaction obtenu

dans le cas ou $R_3'$ représente l'hydrogène, $R_1$, $R_2$, $R_3$ et $R_4$ ayant les significations indiquées en référence à la formule I et X représente l'un des groupes éliminables usuels, en particulier un halogène.

5. Utilisation d'un composé de formule I

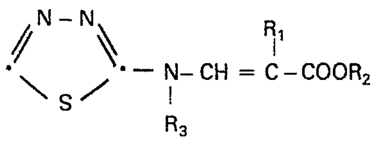

(I)

dans laquelle

$R_1$ représente l'hydrogène ou un groupe alkyle en $C_1$–$C_4$ et

$R_2$ représente un groupe alkyle en $C_1$–$C_4$;

$R_3$ représente un groupe alcoxyalkyle, cycloalkyle en $C_3$–$C_6$ un groupe alkyle en $C_1$–$C_4$ ou alcényle en $C_2$–$C_4$ non substitué ou mono- ou polysubstitué par des halogènes, ou un groupe furyle, thiényle, pyridyle ou phényle non substitué ou mono- à tétra- substitué par des groupes alkyles en $C_1$–$C_4$, des halogènes, des groupes cyano, nitro, alcoxy, alkylthio, alkylsulfonyle ou amino, pour limiter la prolifération des bactéries phytophatogènes, caractérisé en ce que l'on applique la substance active sur l'habitat des bactéries.

6. Utilisation selon la revendication 5, caractérisé en ce que le symbole $R_3$ représente l'hydrogène et les autres symboles ont les significations indiquées en référence à la formule I.

7. Utilisation selon la revendication 5, caractérisé en ce que l'on utilise un composé de formule I du groupe

3-(N-1,3,4-thiadiazolyl-2)-amino-2-méthyl-acrylate de méthyle,

3-(N-1,3,4-thiadiazolyl-2)-amino-2-méthyl-acrylate d'éthyle,

3-(N-1,3,4-thiadiazolyl-2)-amino-2-méthyl-acrylate de n-propyle

3-(N-1,3,4-thiadiazolyl-2)-amino-2-éthyl-acrylate d'éthyle,

3-(N-1,3,4-thiadiazolyl-2)-amino-2-isopropyl-acrylate de méthyle.

8. Utilisation selon la revendication 5, caractérisée en ce que les bactéries appartiennent aux espèces Xanthomonas.

**Claims for the contracting states: BE, CH, LI, DE, FR, GB, IT, NL**

1. A compound of the formula I

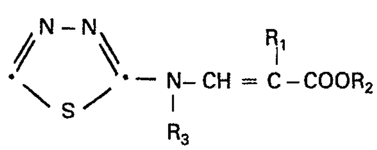

(I)

wherein $R_1$ is hydrogen or $C_1$–$C_4$alkyl, $R_2$ is $C_1$–$C_4$alkyl, $R_3$ is hydrogen or the groupe $-CO-R_4$, in which $R_4$ is alkoxyalkyl, $C_3$–$C_6$cycloalkyl, or is $C_1$–$C_4$alkyl or $C_2$–$C_4$alkenyl each of which is unsubstituted or mono- or polysubstituted by halogen, or is furyl, thienyl, pyridyl or phenyl, each of which is unsubstituted or mono- to tetrasubstituted by $C_1$–$C_4$alkyl, halogen, cyano, nitro, alkoxy, alkylthio, alkylsulfonyl or amino.

2. A compound of the formula I according to claim 1, wherein $R_3$ is hydrogen.

3. A compound selected from the group consisting of

methyl 3-(N-1,3,4-thiadiazolyl-2)-amino-2-methylacrylate,

ethyl 3-(N-1,3,4-thiadiazolyl-2)-amino-2-methylacrylate,

n-propyl 3-(N-1,3,4-thiadiazolyl-2)-amino-2-methylacrylate,

ethyl 3-(N-1,3,4-thiadiazolyl-2)-amino-2-ethylacrylate and

methyl 3-(N-1,3,4-thiadiazolyl-2)-amino-2-isopropylacrylate.

4. A process for the manufacture of a compound of the formula I, which process comprises either

a) condensing 2-amino-1,3,4-thiadiazole of the formula II

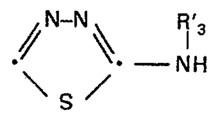

(II)

wherein $R_3'$ is hydrogen, with a compound of the formula III

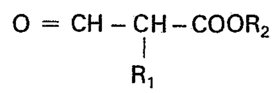

(III)

with elimination of water, and subsequently, if desired, N-acylating the condensate with an acid $R_4COOH$ or preferably with a reactive acid derivative, or

b) reacting a compound of the formula II, wherein $R_3'$ has the same meaning as $R_3$, with a compound of the formula IV

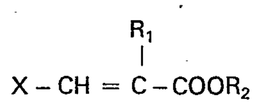

(IV)

preferably in the presence of a base and with the elimination of a compound of the formula H–X, and, if desired, N-acylating the reaction product if $R_3'$ is hydrogen, as described in a), while $R_1$, $R_2$, $R_3$ and $R_4$ are as defined for formula I and X is one of the customary leaving groups, especially halogen.

5. A composition for controlling and/or preventing attack by phytopathogenic bacteria, said composition containing, as active component, at least one compound of the formula I according to claim 1 together with one or more carriers or diluents which are tolerated by plants.

6. A composition according to claim 5 which contains, as active component, a compound of the formula I according to claim 2.

7. A composition according to claim 5 which contains, as active component, a compound of the formula I according to claim 3.

8. A method of controlling phytopathogenic bacteria at a locus, which comprises applying to

said locus a bacterially effective amount of a compound of the formula I according to claim 1.

9. A method according to claim 8 which comprises the use of a compound of the formula I according to claim 2.

10. A method according to claim 8 which comprises the use of a compound of the formula I according to claim 3.

11. A method according to claim 8, wherein the bacteria to be controlled are Xanthomonas species.

**Claims for the contracting state: AT**

1. A compound for control controlling and/or preventing attack by phytopathogenic bacteria, which composition contains, as active component, at least one compound of the formula

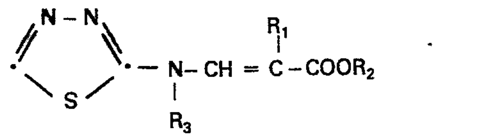

(I)

wherein $R_1$ is hydrogen or $C_1$–$C_4$alkyl, $R_2$ is $C_1$–$C_4$alkyl, $R_3$ is hydrogen or the group –CO–$R_4$, in which $R_4$ is alkoxyalkyl, $C_3$–$C_6$cycloalkyl, or is $C_1$–$C_4$alkyl or $C_2$–$C_4$alkenyl each of which is unsubstituted or mono- or polysubstituted by halogen, or is furyl, thienyl, pyridyl or phenyl, each of which is unsubstituted or mono- to tetrasubstituted by $C_1$–$C_4$alkyl, halogen, cyano, nitro, alkoxy, alkylthio, alkylsulfonyl or amino, together with one or more carriers or diluents which are tolerated by plants.

2. A composition according to claim 1, which contains, as active component, at least one compound of the formula I, wherein $R_3$ is hydrogen and the other substituents are as defined for formula I.

3. A composition according to claim 1, which contains, as active component, at least one compound selected from the group consisting of
methyl 3-(N-1,3,4-thiadiazolyl-2)-amino -2-methylacrylate,
ethyl 3-(N-1,3,4-thiadiazolyl-2)-amino-2-methylacrylate,
n-propyl 3-(N-1,3,4-thiadiazolyl-2)-amino-2-methylacrylate,
ethyl 3-(N-1,3,4-thiadiazolyl-2)-amino-2-ethylacrylate and
methyl 3-(N-1,3,4-thiadiazolyl-2)-amino-2-isopropylacrylate.

4. A process for the manufacture of a compound of the formula I

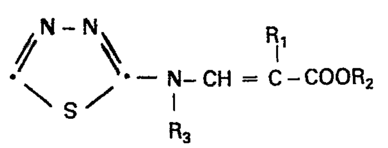

(I)

wherein $R_1$ is hydrogen or $C_1$–$C_4$alkyl, $R_2$ is $C_1$–$C_4$alkyl, $R_3$ is hydrogen or the group –CO–$R_4$, in which $R_4$ is alkoxyalkyl, $C_3$–$C_6$cycloalkyl, or is $C_1$–$C_4$alkyl or $C_2$–$C_4$alkenyl each of which is unsubstituted or mono- or polysubstituted by halogen, or is furyl, thienyl, pyridyl or phenyl, each of which is unsubstituted or mono- to tetrasubstituted by $C_1$–$C_4$alkyl, halogen, cyano, nitro, alkoxy, alkylthio, alkylsulfonyl or amino, which process comprises either

a) condensing 2-amino-1,3,4-thiadiazole of the formula II

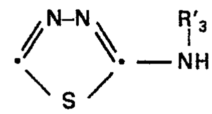

(II)

wherein $R_3'$ is hydrogen, with a compound of the formula III

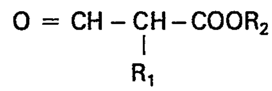

(III)

with elimination of water, and subsequently, if desired, N-acylating the condensate with an acid $R_4COOH$ or preferably with a reactive acid derivative, or

b) reacting a compound of the formula II, wherein $R_3'$ has the same meaning as $R_3$, with a compound of the formula IV

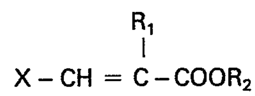

(IV)

preferably in the presence of a base and with the elimination of a compound of the formula H–X, and , if desired, N-acylating the reaction product if $R_3'$ is hydrogen, as described in a), while $R_1$, $R_2$, $R_3$ and $R_4$ are as defined for formula I and X is one of the customary leaving groups.

5. A method of controlling phytopathogenic bacteria at a locus which comprises applying to said locus a bacterially effective amount of a compound of the formula I

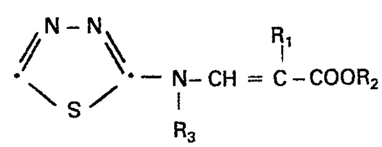

(I)

wherein $R_1$ is hydrogen or $C_1$–$C_4$alkyl, $R_2$ is $C_1$–$C_4$alkyl, $R_3$ is hydrogen or the group –CO–$R_4$, in which $R_4$ is alkoxyalkyl, $C_3$–$C_6$cycloalkyl, or is $C_1$–$C_6$alkyl or $C_2$–$C_4$alkenyl each of which is unsubstituted or mono- or polysubstituted by halogen, or is furyl, thienyl, pyridyl or phenyl, each of which is unsubstituted or mono- to tetrasubstituted by $C_1$–$C_4$alkyl, halogen, cyano, nitro, alkoxy, alkylthio, alkylsulfonyl or amino.

6. A method according to claim 5, wherein the substituent $R_3$ is hydrogen and the other substituents are as defined for formula I.

7. A method according to claim 5, which comprises the use of a compound of the formula I selected from the group consisting of
methyl 3-(N-1,3,4-thiadiazolyl-2)-amino-2-methylacrylate,

ethyl 3-(N-1,3,4-thiadiazolyl-2)-amino-2-methylacrylate,
n-propyl 3-(N-1,3,4-thiadiazolyl-2)-amino-2-methylacrylate,
ethyl 3-(N-1,3,4-thiadiazolyl-2)-amino-2-ethylacrylate and
methyl 3-(N-1,3,4-thiadiazolyl-2 -)-amino-2-isopropylacrylate.

8. A method according to claim 5, wherein the bacteria to be controlled are Xanthomonas species.